# EUROPÄISCHE PATENTSCHRIFT

(11) **EP 2 965 698 B1**
(45) Veröffentlichungstag und Bekanntmachung des Hinweises auf die Patenterteilung: **04.01.2017**
(21) Anmeldenummer: 15176133.5
(22) Anmeldetag: 09.07.2015
(51) Int. Cl.: A61B 17/29, A61B 17/00, A61B 34/30, A61B 90/00

(54) **ENDOSKOPISCHES INSTRUMENT**
ENDOSCOPIC INSTRUMENT
INSTRUMENT ENDOSCOPIQUE

(30) Priorität: 10.07.2014 DE 102014109663
(43) Veröffentlichungstag der Anmeldung: 13.01.2016
(73) Patentinhaber: Karl Storz GmbH & Co. KG, 78532 Tuttlingen (DE)
(72) Erfinder: Blase, Bastian, 10439 Berlin (DE); Röthig, Sebastian, 10553 Berlin (DE); Schlegel, Sebastian, 10825 Berlin (DE)
(74) Vertreter: Witte, Weller & Partner Patentanwälte mbB

(56) Entgegenhaltungen:
- EP-A1- 1 946 704
- EP-A1- 2 322 076
- EP-A2- 2 752 166
- DE-A1-102006 028 001
- US-A1- 2008 065 116
- US-A1- 2011 196 407
- US-B2- 8 574 243

## Beschreibung

Die vorliegende Erfindung betrifft ein endoskopisches Instrument mit einem Schaft, der sich zwischen einem proximalen Ende des Instruments und einem distalen Ende des Instruments entlang einer Längsrichtung erstreckt, wobei am distalen Ende ein Arbeitsabschnitt angeordnet ist und am proximalen Ende ein Betätigungselement angeordnet ist, das eine Betätigung des Arbeitsabschnitts ermöglicht, wobei der Arbeitsabschnitt und das Betätigungselement über ein Schubelement wirkverbunden sind, das sich entlang der Längsrichtung erstreckt und wobei der Arbeitsabschnitt ein Maulstück mit einem ersten Maulteil und einem zweiten Maulteil aufweist, das an einem Maulteilhalter verschwenkbar angeordnet ist.

Die Anzahl an chirurgischen Eingriffen, die mittels endoskopischer Operationstechniken durchgeführt werden, ist in den vergangenen Jahren immer weiter gestiegen. Dies ist einerseits darin begründet, dass immer mehr Operationstechniken entwickelt worden sind, die einen weniger traumatischen Eingriff am Patienten ermöglichen als bei konventionellen Operationstechniken. Es sind aber außerdem auch immer weiter verfeinerte endoskopische Instrumente entwickelt worden, die aufgrund eines geringeren Durchmessers ein geringeres Trauma beim Patienten verursachen und dem Benutzer mehr Freiheitsgrade während der Operation einräumen, wenn sich das endoskopische Instrument im Körper des Patienten befindet.

Trotz dieser vorteilhaften Entwicklungen bestehen heute immer noch Schwierigkeiten darin, ein endoskopisches Instrument mit einem möglichst geringen Durchmesser bereitzustellen, dessen Arbeitsabschnitt am distalen Ende des Instruments dem Benutzer aber dennoch eine große Positionierfreiheit und Beweglichkeit erlaubt. Dabei wäre es insbesondere hilfreich, wenn in einem endoskopischen Instrument mehrere Arbeitsabschnitte verwendet werden könnten, da dies einen Wechsel der endoskopischen Instrumente oder das Einführen mehrerer endoskopischer Instrumente vermeiden würde.

Beispiele von endoskopischen Instrumenten, die versuchen, eine große Positionierfreiheit und Beweglichkeit zu ermöglichen, sind in den Schriften US 7,686,826, CA 2 663 305 und US 2007/0283970 gezeigt.

EP 2 322 076 A1, welches als nächstliegender Stand der Technik angesehen wird, zeigt ein Endoskop mit Behandlungskanälen, in die jeweils ein flexibler Positionierungsschlauch eingeführt werden kann. In jeden Positionierungsschlauch wiederum kann ein längliches endoskopisches Instrument eingeführt werden. Dabei wird zunächst der Positionierungsschlauch in eine gewünschte Position gebracht und dort gegebenenfalls verriegelt. Dann wird in den Positionierungsschlauch das endoskopische Instrument eingeführt, um an der Operationsstelle zu arbeiten.

EP 1 946 704 A1 zeigt ein endoskopisches Instrument, bei dem durch Betätigung eines Bediendrahts ein Maulteil des endoskopischen Instruments geöffnet oder geschlossen werden kann. Dem Maulteil ist eine Begrenzungseinrichtung zugeordnet, die eine Drehung des Maulteils um seine Längsachse nur über einen vorgegebenen Winkelbereich zulässt.

Es ist daher eine Aufgabe der vorliegenden Erfindung, ein verbessertes endoskopisches Instrument aufzuzeigen. Dabei soll das verbesserte Instrument bei geringem Durchmesser des Schafts dem Benutzer mehrere Freiheitsgrade bei der Betätigung eines Arbeitsabschnitts am distalen Ende des endoskopischen Instruments ermöglichen.

Die Aufgabe wird gelöst durch ein endoskopisches Instrument nach Anspruch 1, mit einem Schaft, der sich zwischen einem proximalen Ende des Instruments und einem distalen Ende des Instruments entlang einer Längsrichtung erstreckt, wobei am distalen Ende ein Arbeitsabschnitt angeordnet ist und am proximalen Ende ein Betätigungselement angeordnet ist, das eine Betätigung des Arbeitsabschnitts ermöglicht, wobei der Arbeitsabschnitt und das Betätigungselement über ein Schubelement wirkverbunden sind, das sich entlang der Längsrichtung erstreckt, wobei der Arbeitsabschnitt ein Maulstück mit einem ersten Maulteil und einem zweiten Maulteil aufweist, das an einem Maulteilhalter verschwenkbar angeordnet ist, wobei der Maulteilhalter im Schaft entlang der Längsrichtung verschiebbar und um die Längsrichtung rotierbar angeordnet ist und das Schubelement mit dem Maulstück gekoppelt ist, wobei das Maulstück derart ausgebildet ist, dass es im geschlossenen Zustand in den Schaft einfahrbar ist, wobei in dem Instrument eine Sperrvorrichtung angeordnet ist, die dafür ausgebildet ist, ein Einfahren des Maulstücks in den Schaft in einem auf die Längsrichtung bezogenen vorgegebenen Winkelbereich zu blockieren.

Gemäß der Erfindung ist das Schubelement entlang der Längsrichtung zumindest dann relativ zum Maulteilhalter verlagerbar, wenn das Maulstück aus dem Schaft ausgefahren ist.

Diese Ausgestaltung ermöglicht es, dass auf konstruktiv einfache Weise durch das Schubelement das Öffnen und das Schließen des Maulstücks bewirkt werden kann.

Gemäß der Erfindung übt das Schubelement bei einem Schub auf das distale Ende hin eine Kraft auf das Maulstück aus, die das Maulstück öffnen kann, und ist der Schaft räumlich so ausgebildet, dass ein Öffnen des Maulstücks im Schaft verhindert wird.

Diese Ausgestaltung ermöglicht es, dass das Schubelement zwar primär mit dem Maulstück zusammenwirkt, die Wirkung von dem Maulstück aber auf den Maulteilhalter übertragen wird, wenn sich das Maulstück nicht öffnen kann. Durch einen Schub auf das Schubelement wirkt primär eine Kraft, die das Maulstück öffnen kann. Wird das Maulstück jedoch im Schaft daran gehindert, sich zu öffnen, so wird diese Kraft auf den Maulteilhalter übertragen, an dem das Maulstück verschwenkbar angeordnet ist. Dies wiederum führt dazu, dass der Schub auf das Schubelement nun auf den Maulteilhalter wirkt und den Maulteilhalter im Schaft verlagert.

Eine Besonderheit des vorliegenden endoskopischen Instruments ist es, dass es möglich ist, mit einem einzigen Schubelement das Maulstück aus dem Schaft in eine Arbeitsposition herauszufahren, das Maulstück zu öffnen und zu schließen, das Maulstück um die Längsrichtung zu rotieren und das Maulstück in den Schaft in eine Ruheposition einzufahren. Zwar ist es auch möglich, diese verschiedenen Funktionen über zwei oder mehr Schubelemente zu steuern, doch wird in der Verwendung genau eines Schubelements ein Vorteil in dem besonders geringen Durchmesser des endoskopischen Instruments gesehen.

Eine weitere Besonderheit des endoskopischen Instruments wird darin gesehen, dass mittels der Sperrvorrichtung beeinflusst werden kann, ob eine Betätigung des Schubelements ein Öffnen oder Schließen des Maulteils bewirken soll oder ein Einfahren oder Ausfahren des Maulteils relativ zum Schaft bewirken soll. Dies wird dadurch erreicht, indem die Sperrvorrichtung in einem ersten Zustand ein Einfahren des Maulstücks in den Schaft verhindert und in einem zweiten Zustand ein Einfahren des Maulstücks in den Schaft ermöglicht.

Bei einer bevorzugten Ausgestaltung ist die Funktionsweise konkret wie folgt: Wenn sich die Sperrvorrichtung in dem ersten Zustand befindet und ein Zug auf das Schubelement ausgeübt wird, also eine Kraft in Richtung auf das proximale Ende des endoskopischen Instruments hin, kann dieser Zug nicht das Einfahren des Maulstücks in den Schaft bewirken, da die Sperrvorrichtung das Einfahren blockiert. Stattdessen wirkt die Kraft auf das Maulstück und führt zu einer Betätigung des Maulstücks. Bei einer bevorzugten Ausgestaltung führt ein Zug am Schubelement zu einem Schließen des Maulstücks.

Wenn sich die Sperrvorrichtung dagegen im zweiten Zustand befindet, führt ein Zug am Schubelement dazu, dass das Maulstück in den Schaft eingefahren wird. Bei einer bevorzugten Ausführungsform ist das Maulstück dabei so ausgebildet, dass es beim Einfahren in den Schaft geschlossen wird. Eine andere bevorzugte Ausführungsform erfordert dagegen ein vorheriges Schließen des Maulstücks durch den Benutzer. Dafür ist das Maulstück dann so ausgestaltet, dass es ein Einfahren des Maulstücks im geöffneten Zustand selbst blockiert. Für ein Einfahren des Maulstücks ist es in diesem Fall erforderlich, das Maulstück zunächst zu schließen, und die Sperrvorrichtung dann in den zweiten Zustand zu überführen, sofern dies nicht bereits erfolgt ist.

Die Überführung der Sperrvorrichtung vom ersten Zustand in den zweiten Zustand bzw. von dem zweiten Zustand in den ersten Zustand erfolgt bevorzugt über eine Rotation des Schubelements. Es ist zwar auch möglich, die Einstellung der Sperrvorrichtung mittels eines zusätzlichen Steuerelements zu realisieren. Es wird im Hinblick auf einen geringen Durchmesser des endoskopischen Instruments aber als vorteilhaft angesehen, wenn die Einstellung mittels eines bereits vorhandenen Elements vorgenommen werden kann, hier dem Schubelement.

Bei einer bevorzugten Ausgestaltung rotiert der Benutzer das Schubelement - und damit auch das Maulstück - in eine bestimmte Winkelposition. Diese Winkelposition ist davon abhängig, ob der Benutzer das Einfahren des Maulstücks blockieren oder freigeben möchte. Mit anderen Worten, rotiert der Benutzer das Maulstück in eine Winkelposition, die sich in dem vorgegeben Winkelbereich befindet, so ist das Einfahren des Maulstücks in den Schaft blockiert. Rotiert der Benutzer das Maulstück in eine Winkelposition, die außerhalb des vorgegebenen Winkelbereichs liegt, so wird das Einfahren des Maulstücks in den Schaft ermöglicht.

Bei einer bevorzugten Ausgestaltung wird genau ein zusammenhängender Winkelbereich verwendet, in dem die Sperrvorrichtung das Einfahren des Maulstücks blockiert. Bei anderen bevorzugten Ausgestaltungen sind zwei oder mehr Winkelbereiche ausgebildet, in denen die Sperrvorrichtung das Einfahren des Maulstücks blockiert. Dabei wird es als vorteilhaft angesehen, wenn der Winkelbereich bzw. die Summe der Winkelbereiche, in denen das Einfahren blockiert wird, größer ist als die verbleibenden Winkelbereiche, in denen das Einfahren möglich ist.

Bei einer bevorzugten Ausgestaltung besteht keine direkte mechanische Wirkverbindung zwischen dem Schubelement und dem Maulteilhalter. Insbesondere findet keine direkte Kraftübertragung vom Schubelement auf den Maulteilhalter statt. Stattdessen findet bei einer bevorzugten Ausgestaltung die Kraftübertragung vom Schubelement über das Maulstück auf den Maulteilhalter statt.

Dies ermöglicht es, eine über das Schubelement übertragene Kraft zunächst an das Maulstück zu leiten. Wenn das Maulstück die Kraft für ein Öffnen oder Schließen des Maulstücks aufnehmen kann, kann der Maulteilhalter im Wesentlichen in Ruhe bleiben. Kann das Maulstück die Kraft nicht in eine Bewegung umsetzen, z.B. weil das Maulstück geschlossen ist, so kann die Kraft an den Maulteilhalter weitergeleitet werden und z.B., in Abhängigkeit vom Zustand der Sperrvorrichtung, ein Einfahren des Maulstücks bewirken.

Es sei darauf hingewiesen, dass der Begriff Schub sowohl einen positiven Schub als auch einen negativen Schub, auch Zug genannt, umfasst. Das Schubelement überträgt also sowohl einen Schub als auch einen Zug. Ferner sei darauf hingewiesen, dass der Begriff des Verschwenkens des Maulstücks insbesondere ein Öffnen und Schließen des Maulstücks umfasst.

Damit ist die Aufgabe vollständig gelöst.

Bei einer vorteilhaften Ausgestaltung weist die Sperrvorrichtung ein erstes Sperrelement auf, das ortsfest relativ zum Schaft angeordnet ist, und weist die Sperrvorrichtung ein zweites Sperrelement auf, das ortsfest relativ zum Maulteilhalter angeordnet ist, wobei das erste Sperrelement bevorzugt als Nut und das zweite Sperrelement bevorzugt als Vorsprung ausgestaltet sind.

Diese Ausgestaltung kann fertigungstechnisch vorteilhaft sein. Es besteht außerdem die Möglichkeit, den Durchmesser des endoskopischen Instruments gering zu halten. Wenn sich das erste Sperrelement und das zweite Sperrelement in Bezug auf die Längsrichtung, also bezüglich eines Schubs oder Zugs am Schubelement, gegenseitig blockieren, ist dadurch auch die Bewegung des Maulteilhalter relativ zum Schaft blockiert. Kann sich das erste Sperrelement relativ zum zweiten Sperrelement in Längsrichtung verlagern, so kann sich auch der Maulteilhalter relativ zum Schaft verlagern, und ein Einfahren des Maulstücks in den Schaft ist möglich. Anhand der Ausführungsbeispiele wird noch verdeutlicht, wie die Verwendung einer Nut als erstes Sperrelement und eines Vorsprungs als zweites Sperrelement vorteilhaft zur Realisierung der Sperrvorrichtung eingesetzt werden können. Es sei aber bereits an dieser Stelle darauf hingewiesen, dass grundsätzlich auch die konstruktive Umkehr möglich ist, sprich, wenn das erste Sperrelement als Vorsprung und das zweite Sperrelement als Nut ausgebildet ist, da es auf das gegenseitige Sperren von erstem und zweitem Sperrelement in Längsrichtung ankommt und nicht auf die spezielle Art und Weise wie die Sperrung bewirkt wird.

Bei einer weiteren vorteilhaften Ausgestaltung weist die Sperrvorrichtung eine Längsnut auf, die im Schaft entlang der Längsrichtung ausgebildet ist, und weist die Sperrvorrichtung einen Vorsprung auf, der am Maulteilhalter angeordnet ist und bei einer Verlagerung des Maulteilhalters entlang der Längsrichtung in der Längsnut geführt ist.

Diese Ausgestaltung ermöglicht eine einfache Konstruktion der Sperrvorrichtung. Die Längsnut kann dabei bezogen auf die Längsrichtung des endoskopischen Instrument sehr kurz ausgeführt sein. Die Längsnut hat bei einer bevorzugten Ausgestaltung die Funktion, dass ein Einfahren des Maulstücks in den Schaft nur dann möglich ist, wenn der Vorsprung in die Längsnut eintritt. Mit anderen Worten gelingt ein Einfahren des Maulstücks in den Schaft nur dann, wenn der Vorsprung an dem Maulteilhalter bei einem Zug am Schubelement in die Längsnut eingeführt wird. Verfehlt der Vorsprung den Eintritt in die Längsnut, also die Stirnseite der Längsnut, verhindert der Vorsprung ein Einfahren des Maulstücks.

Bei einer weiteren vorteilhaften Ausgestaltung ist am distalen Ende des Schafts eine Ringnut ausgebildet, in die ein am Maulteilhalter angeordneter Vorsprung eintreten kann, so dass der Maulteilhalter um die Längsrichtung rotiert werden kann, wenn der Vorsprung in die Ringnut eingetreten ist.

Diese Ausgestaltung ermöglicht es, dass das Maulstück zumindest dann um die Längsrichtung rotiert werden kann, wenn der Vorsprung in die Ringnut eingetreten ist. Dabei ist es vorteilhaft, wenn die Ringnut an einer dem proximalen Ende des Instruments zugewandten Seite eine Öffnung aufweist. Befindet sich der Vorsprung bei seiner Rotation dann vor dieser Öffnung, kann mittels eines Zugs am Schubelement der Maulteilhalter mit dem Vorsprung in Richtung auf das proximale Ende gezogen und damit das Maulstück eingefahren werden. Der Winkelbereich, in dem die Öffnung nicht angeordnet ist, ist dann der vorhergenannte Winkelbereich, in dem ein Einfahren des Maulstücks blockiert wird. Bei einer bevorzugten Ausgestaltung stellt die Öffnung in der Ringnut den Beginn einer Längsnut dar, insbesondere einer Längsnut, wie sie zuvor beschrieben wurde.

Bei einer weiteren vorteilhaften Ausgestaltung weist die Sperrvorrichtung eine Längsnut auf, die im Schaft entlang der Längsrichtung ausgebildet ist, weist die Sperrvorrichtung eine Ringnut am distalen Ende des Schafts auf und weist die Sperrvorrichtung einen am Maulteilhalter angeordneten Vorsprung auf, wobei der Vorsprung in der Längsnut und in der Ringnut geführt ist.

Diese Ausgestaltung ermöglicht eine konstruktiv einfache Realisierung der Sperrvorrichtung. Die Längsnut mündet dabei in die Ringnut, so dass der Vorsprung von der Längsnut in die Ringnut und von der Ringnut in die Längsnut überführt werden kann. Solange sich der Vorsprung in der Ringnut befindet, kann der Maulteilhalter mit dem Maulstück um die Längsrichtung rotiert werden. Wenn sich der Vorsprung an einer Stelle entlang der Umfangserstreckung der Ringnut befindet, die nicht mit dem Übergang der Längsnut in die Ringnut übereinstimmt, blockiert der Vorsprung eine Bewegung des Maulteilhalters in Längsrichtung. Dies bedeutet, dass sich das Maulstück bei einem Zug auf das Schubelement schließt und bei einem Schub auf das Schubelement öffnet. Wenn sich der Vorsprung an einer Stelle befindet, an der die Längsnut in die Ringnut mündet, führt ein Zug auf das Schubelement zu einem Einfahren des Maulstücks. Der Vorsprung wird nicht zeitgleich in Längsnut und Ringnut geführt.

Bei einer weiteren vorteilhaften Ausgestaltung der Erfindung sind das erste Maulteil und das zweite Maulteil um eine gemeinsame erste Achse verschwenkbar und ist das Maulstück an einer zweiten Achse mit dem Schubelement gekoppelt, wobei die erste Achse und die zweite Achse voneinander beabstandet sind und insbesondere die erste Achse weiter distal liegt als die zweite Achse.

Diese Ausgestaltung ermöglicht eine konstruktiv vorteilhafte Gestaltung des Maulstücks. Insbesondere kann erreicht werden, dass auf das Maulstück ausreichend große Kräfte für einen Arbeitsvorgang oder Arbeitsschritt aufgebracht werden können.

Bei einer weiteren vorteilhaften Ausgestaltung der Erfindung ist das erste Maulteil über eine erste gelenkige Verbindung mit dem Schubelement gekoppelt und/oder ist das zweite Maulteil über eine zweite gelenkige Verbindung mit dem Schubelement gekoppelt.

Diese Ausgestaltung ermöglicht es auch bei räumlich geringen Abmessungen, dass das Maulstück ausreichend weit geöffnet und mit ausreichend Kraft geschlossen werden kann.

Bei einer weiteren vorteilhaften Ausgestaltung ist am distalen Ende des Schafts ein Anschlag ausgebildet, der eine Verlagerung des Maulteilhalters entlang der Längsrichtung distalseitig begrenzt.

Diese Ausgestaltung kann bewirken, dass bei einem Schub auf das Schubelement das Maulstück im Wesentlichen im geschlossenen Zustand aus dem Schaft ausgefahren wird und sich erst dann öffnet, wenn der Maulteilhalter an dem Anschlag anschlägt. Dies kann es in besonders guter Weise dem Benutzer ermöglichen, die Verlagerung des Maulstücks und dessen Öffnung nacheinander zu kontrollieren, auch wenn die Verlagerung und Öffnung des Maulstücks über ein einzelnes Schubelement erfolgt. Wenn sich der Maulteilhalter distalseitig nicht weiter verlagern kann, wird die mittels des Schubs auf das Schubelement übertragene Kraft auf das Maulstück übertragen, das sich dann öffnet.

Bei einer weiteren bevorzugten Ausgestaltung weist das Schubelement einen länglichen Schubschaft auf, der in einer Hülse des Maulteilhalters verschiebbar geführt ist, wobei die Hülse in einem Kanal im Inneren des Schafts geführt ist.

Diese Ausgestaltung ermöglicht eine gute Übertragung eines Zugs bzw. eines Schubs mittels des Schubelements, selbst wenn der Schaft des endoskopischen Instruments lang ist und/oder der Durchmesser des Schafts klein ist.

Bei einer weiteren vorteilhaften Ausgestaltung weist der Schaft am distalen Ende einen Bereich auf, der abwinkelbar ist, und wird die Abwinklung von einem Abwinklungssteuerelement gesteuert, das vom proximalen Ende des Instruments betätigbar ist.

Diese Ausgestaltung stellt einen weiteren Freiheitsgrad zur Verfügung, mit dem das Maulstück verlagert werden kann.

Bei einer weiteren vorteilhaften Ausgestaltung ist am distalen Ende ein Arbeiten mit zwei Arbeitsabschnitten möglich, wobei am proximalen Ende ein zweites Betätigungselement angeordnet ist, das eine Betätigung des zweiten Arbeitsabschnitts ermöglicht, wobei der zweite Arbeitsabschnitt und das zweite Betätigungselement über ein zweites Schubelement wirkverbunden sind, das sich entlang der Längsrichtung erstreckt, wobei der zweite Arbeitsabschnitt ein zweites Maulstück mit einem dritten Maulteil und einem vierten Maulteil aufweist, das an einem zweiten Maulteilhalter verschwenkbar angeordnet sind, wobei der zweite Maulteilhalter im Schaft entlang der Längsrichtung verschiebbar und um die Längsrichtung rotierbar angeordnet ist und das zweite Schubelement mit dem zweiten Maulstück gekoppelt ist, wobei das zweite Maulstück derart ausgebildet ist, dass es im geschlossenen Zustand in den Schaft einfahrbar ist, wobei innerhalb des Instruments eine zweite Sperrvorrichtung angeordnet ist, die dafür ausgebildet ist, ein Einfahren des zweiten Maulstücks in den Schaft in einem auf die Längsrichtung bezogenen zweiten Winkelbereich zu blockieren.

Diese Ausgestaltung ermöglicht es, mit zwei oder mehr Arbeitsabschnitten zu arbeiten, wobei die Ausgestaltung nach Bedarf so gewählt werden kann, dass stets nur mit einem ausgefahrenen Arbeitsabschnitt gearbeitet wird, oder zwei oder mehr Arbeitsabschnitten gleichzeitig ausgefahren sein können und ggf. auch mit zwei oder mehr Arbeitsabschnitten gleichzeitig gearbeitet werden kann. Es ist zudem möglich, auch weitere Arbeitsabschnitte zu verwenden, die nicht über ein Maulstück verfügen.

Bei einer weiteren vorteilhaften Ausgestaltung weist der Schaft am distalen Ende einen Öffnungsschlitz auf, in den ein Vorsprung der Sperrvorrichtung bei einer Rotation um die Längsrichtung eintreten kann.

Wie noch anhand eines Ausführungsbeispiels gezeigt wird, ermöglicht diese Ausgestaltung einen geringen Durchmesser des Schafts des endoskopischen Instruments.

Bei einer weiteren vorteilhaften Ausgestaltung beträgt der Winkelbereich mindestens 180°, bevorzugt mindestens 270°, besonders bevorzugt mindestens 315° und insbesondere mindestens 335°.

Diese Ausgestaltung ermöglicht es in Abhängigkeit von der gewünschten Auslegung der Bedienung des endoskopischen Instruments, dass einerseits ein ausreichend großer Bereich für eine Betätigung des Maulstücks gegeben ist und andererseits das Maulstück einfach und zuverlässig eingefahren werden kann.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung näher dargestellt und werden in der nachfolgenden Beschreibung näher erläutert. Es zeigen:
- Fig. 1: eine erste Ausführungsform eines endoskopischen Instruments;
- Fig. 2: eine Vergrößerung des distalen Teils des endoskopischen Instruments gemäß Fig. 1;
- Fig. 3: einen Schnitt durch den distalen Teil gemäß Fig. 2;
- Fig. 4: eine vergrößerte, teilgeschnittene Ansicht in das Innere des distalen Teils des endoskopischen Instruments;
- Fig. 5: vereinfachte Ansichten des Inneren des distalen Teils des endoskopischen Instruments mit verschiedenen Positionen des geschlossenen Maulstücks;
- Fig. 6: einen Teil eines Schafts eines endoskopischen Instruments gemäß einer zweiten Ausführungsform;
- Fig. 7: zeigt die Darstellung aus Fig. 6 mit einem aufgesetzten Abschlusselement;
- Fig. 8: die Anordnung von zwei Arbeitsabschnitten in isolierter Darstellung;
- Fig. 9: die Arbeitsabschnitte aus Fig. 8, die in den Schaft mit Abschlusselement gemäß Fig. 7 eingesetzt sind;
- Fig. 10: eine Hülle des Schafts gemäß Fig. 7;
- Fig. 11: eine Darstellung der Fig. 9 mit der Hülle aus Fig. 10;
- Fig. 12: die Darstellung aus Fig. 11, wobei der Schaft, das Abschlusselement und die Hülle transparent dargestellt sind;
- Fig. 13: eine Ansicht in das Innere des distalen Teils des endoskopischen Instruments gemäß der zweiten Ausführungsform;
- Fig. 14: eine Draufsicht auf das Abschlusselement bei Blickrichtung auf das distale Ende zu;
- Fig. 15: eine perspektivische Darstellung des Abschlusselements gemäß Fig. 14; und
- Fig. 16: eine weitere Ausführungsform des Abschlusselements.

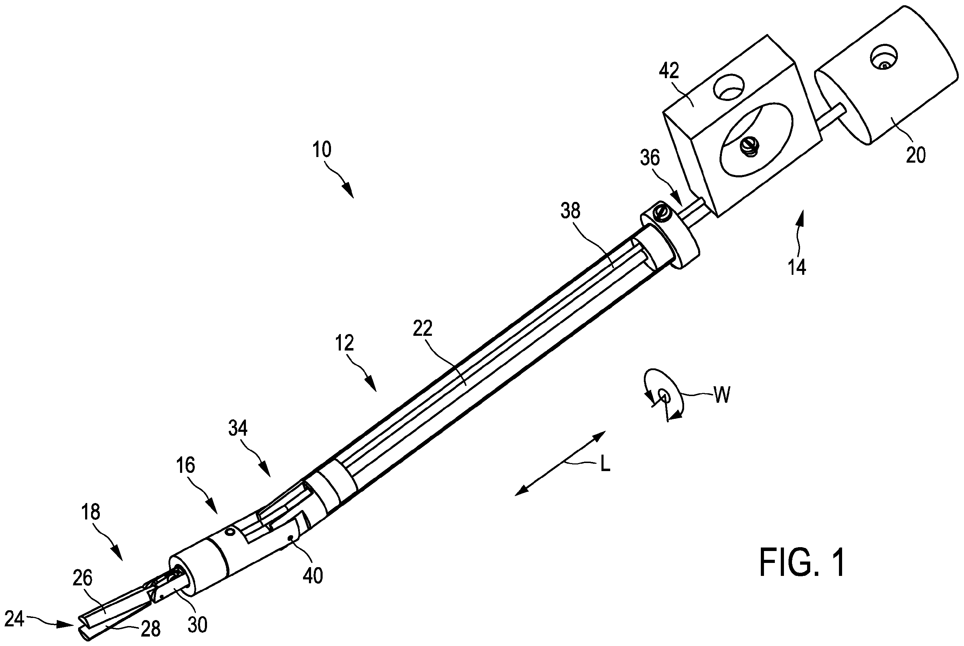

Fig. 1 zeigt ein endoskopisches Instrument 10 mit einem Schaft 12, der sich zwischen einem proximalen Ende 14 des Instruments 10 und einem distalen Ende 16 des Instruments 10 entlang einer Längsrichtung L erstreckt. Am distalen Ende 16 ist ein Arbeitsabschnitt 18 angeordnet, und am proximalen Ende 14 ist ein symbolisch dargestelltes Betätigungselement 20 angeordnet. Das Betätigungselement 20 ermöglicht eine Betätigung des Arbeitsabschnitts 18.

Der Arbeitsabschnitt 18 und das Betätigungselement 20 sind über ein Schubelement 22 miteinander wirkverbunden, das sich entlang der Längsrichtung L erstreckt.

Der Arbeitsabschnitt 18 weist ein Maulstück 24 mit einem ersten Maulteil 26 und einem zweiten Maulteil 28 auf. Das Maulstück 24 ist an einem Maulteilhalter 30 verschwenkbar angeordnet. Der Maulteilhalter 30 ist im Schaft 12 entlang der Längsrichtung L verschiebbar und um die Längsrichtung L rotierbar angeordnet. Das Schubelement 22 ist mit dem Maulstück 24 gekoppelt. Das Maulstück 24 ist derart ausgebildet, dass es zumindest im geschlossenen Zustand in den Schaft 12 einfahrbar ist.

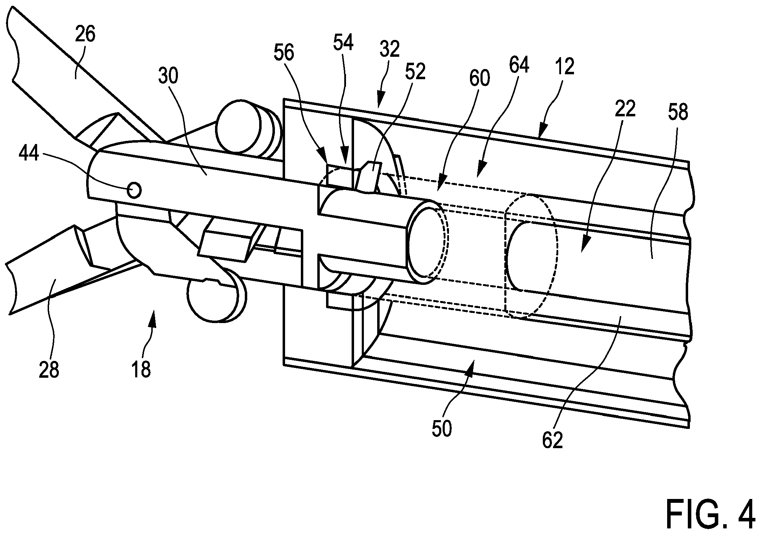

In dem Instrument 10 ist eine Sperrvorrichtung 32 (siehe Fig. 4) angeordnet, die hier durch den Schaft 12 verdeckt ist. Die Sperrvorrichtung 32 ist dafür ausgebildet, ein Einfahren des Maulstücks 24 in den Schaft 12 in einem auf die Längsrichtung L bezogenen vorgegebenen Winkelbereich W zu blockieren.

Der Schaft 12 weist am distalen Ende 16 einen Bereich 34 auf, der abwinkelbar ist. Die Abwinklung des Bereichs 34 wird von einem Abwinklungssteuerelement 36 gesteuert, das vom proximalen Ende 14 des Instruments 10 betätigbar ist. Bei einer bevorzugten Ausgestaltung, hier nicht gezeigt, wird eine Abwinklungseinrichtung gemäß WO 2014/006186 A1 verwendet.

Das Abwinklungssteuerelement 36 weist hier einen Abwinklungsstab 38 auf, der zumindest in der Nähe des einachsigen Gelenks 40 flexibel ausgestaltet ist. Es ist grundsätzlich aber auch möglich, den gesamten Abwinklungsstab flexibel auszuführen und segmentweise mit dünnen Hüllrohren zu versteifen. Um einen Schub bzw. einen Zug auf den Abwinklungsstab 38 auszuüben, ist am proximalen Ende 14 ein symbolisch dargestellter Griff 42 angeordnet.

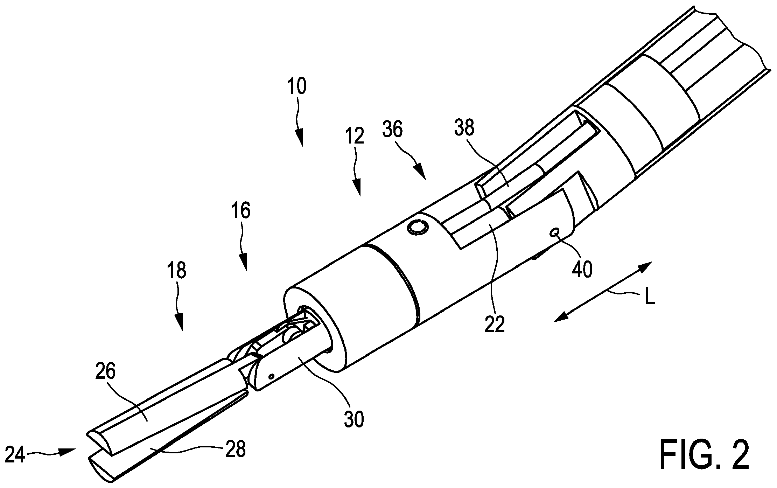

Fig. 2 zeigt eine Vergrößerung des distalen Teils des endoskopischen Instruments 10 aus Fig. 1. Hier und im Nachfolgenden werden bereits eingeführte Bezugszeichen auch in den weiteren Figuren verwendet, um Elemente zu kennzeichnen, die funktional gleichwirken oder zumindest eine vergleichbare Funktion haben.

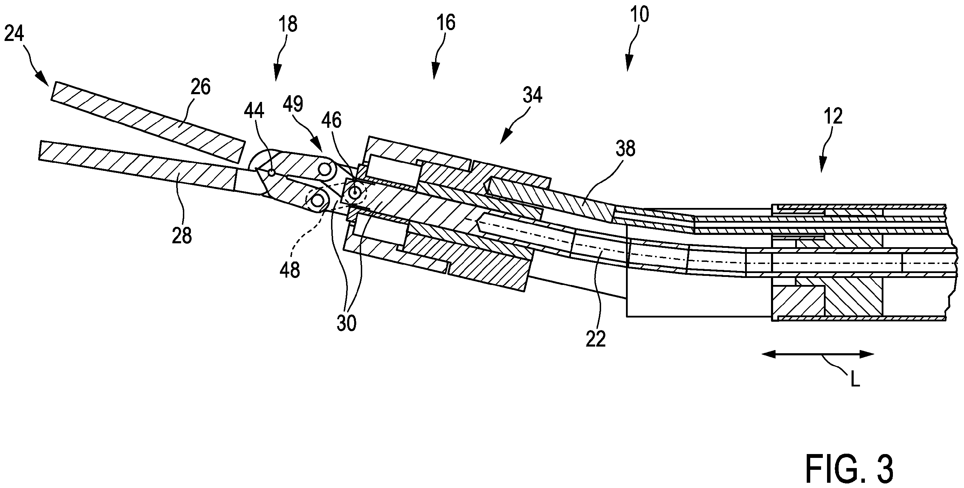

Fig. 3 zeigt eine Schnittansicht durch den distalen Teil des Instruments 10 gemäß Fig. 2. Die Schnittansicht ist vereinfacht und zeigt nur die in Fig. 2 erkennbaren Bauteile.

Das erste Maulteil 26 und das zweite Maulteil 28 sind um eine gemeinsame erste Achse 44 verschwenkbar. Das Maulstück 24 ist an einer zweiten Achse 46 mit dem Schubelement 22 gekoppelt, wobei die erste Achse 44 und die zweite Achse 46 voneinander beabstandet sind. Bei dieser Ausführungsform liegt die erste Achse 44 weiter distal als die zweite Achse 46. Das erste Maulteil 26 ist über eine erste gelenkige Verbindung 48 (gestrichelt gezeichnet) mit dem Schubelement 22 gekoppelt, und das zweite Maulteil 28 ist über eine zweite gelenkige Verbindung 49 mit dem Schubelement 22 gekoppelt.

Fig. 4 zeigt in geschnittener Ansicht einen vergrößerten Ausschnitt des distalen Teils des endoskopischen Instruments 10. Die Bauteile sind teilweise transparent dargestellt, um ein besseres Verständnis dieser Ausführungsform zu ermöglichen.

Es ist nun die Sperrvorrichtung 32 zu erkennen. Die Sperrvorrichtung 32 weist ein erstes Sperrelement auf, das ortsfest relativ zum Schaft 12 angeordnet ist und hier als Längsnut 50 ausgebildet ist. Die Sperrvorrichtung 32 weist außerdem ein zweites Sperrelement auf, das ortsfest relativ zum Maulteilhalter 30 angeordnet ist und hier als Vorsprung 52 ausgestaltet ist.

Am distalen Ende des Schafts 12 ist eine Ringnut 54 ausgebildet, in die der am Maulteilhalter 30 angeordnete Vorsprung 52 eintreten kann, so dass der Maulteilhalter 30 um die Längsrichtung L rotiert werden kann, wenn der Vorsprung 52 in die Ringnut 54 eingetreten ist. Die Längsnut 50 mündet in die Ringnut 54.

Am distalen Ende des Schafts 12 ist ein Anschlag 56 ausgebildet, der eine Verlagerung des Maulteilhalters 30 entlang der Längsrichtung L distalseitig begrenzt. Das Schubelement 22 ist entlang der Längsrichtung L relativ zum Maulteilhalter 30 verlagerbar, wenn das Maulstück 24, wie gezeigt, aus dem Schaft 12 ausgefahren ist. Bei dieser bevorzugten Ausführungsform ist der Anschlag 56 die distale Seitenwand der Ringnut 54.

Das Schubelement 22 weist einen länglichen Schubschaft 58 auf, der in einer Hülse 60 des Maulteilhalters 30 verschiebbar geführt ist. Die Hülse 60 ist hier in einem Kanal 62 im Inneren des Schafts 12 geführt.

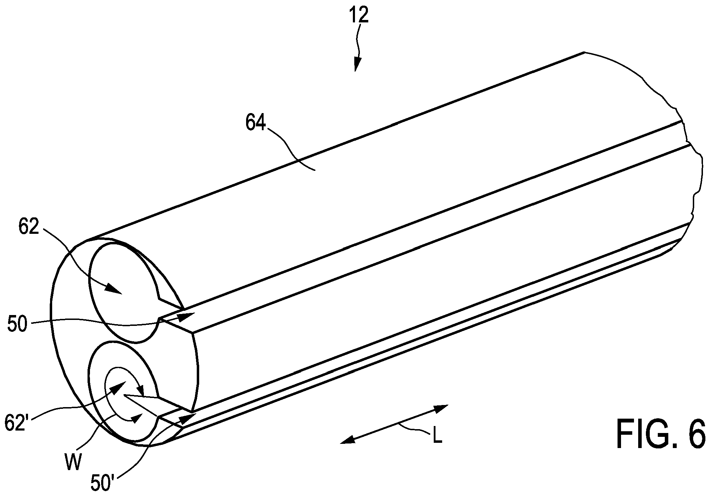

Bei der hier gezeigten Ausführungsform ist der Kanal 62 in einem Zuführelement 64 ausgebildet, in dem auch die Längsnut 50 ausgebildet ist. Dies wird in Fig. 6 unter Bezugnahme auf ein weiteres Ausführungsbeispiel noch verdeutlicht werden.

Schließlich zeigt Fig. 4 auch ein Abschlusselement 66, in dem die Ringnut 54 ausgebildet ist. Bei dieser Ausführungsform ist am Abschlusselement 66 auch der Anschlag 56 ausgebildet.

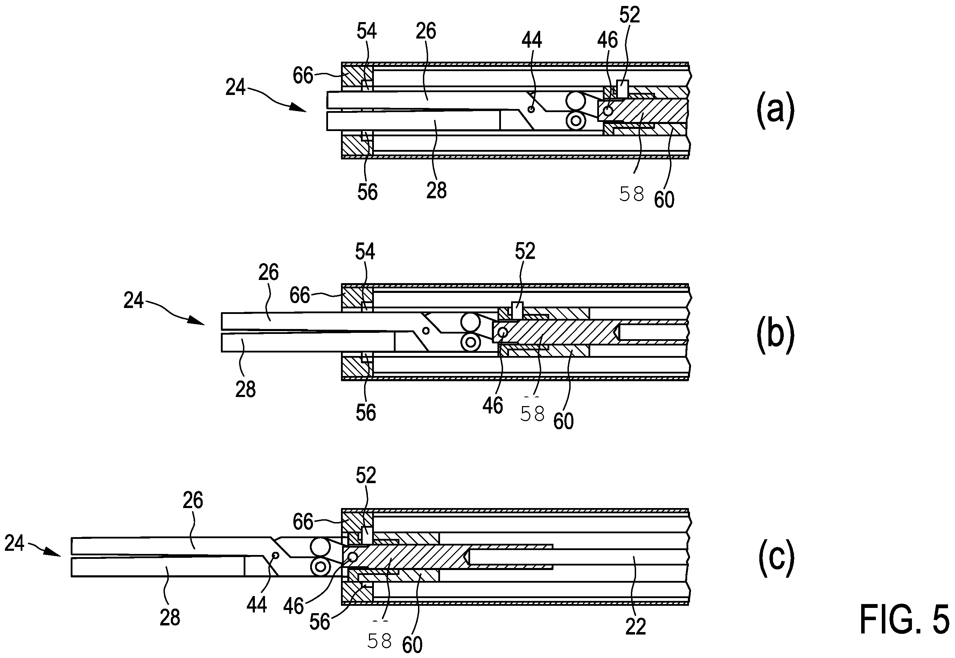

Fig. 5 zeigt eine Darstellung des Maulstücks 24 in verschiedenen Positionen relativ zum Schaft 12. Dabei zeigt der Teil (a) den eingefahrenen Zustand des Maulstücks 24 und Teil (b) den teilweise ausgefahrenen Zustand des Maulstücks 24. Der Teil (c) zeigt schließlich den ganz ausgefahrenen Zustand des Maulstücks 24, wobei der Vorsprung 52 von der Längsnut 50 in die Ringnut 54 eingetreten ist und der Anschlag 56 eine weitere distalseitige Verlagerung des Maulteilhalters 30 verhindert.

Die räumliche Ausgestaltung des Schafts 12, genauer gesagt des Zuführelements 64, ist so gewählt, dass sich das Maulstück 24 im Schaft nicht öffnen kann. Das Schubelement 22 überträgt bei einem Schub zwar eine Kraft auf das Maulstück 24, die das Maulstück 24 öffnen könnte. Der Schaft 12 ist aber räumlich so ausgebildet, dass das Öffnen des Maulstücks 24 im Schaft 12 verhindert wird.

Die nachfolgenden Ausführungen beziehen sich auf eine zweite Ausführungsform des endoskopischen Instruments 10, das die Verwendung von zwei Arbeitsabschnitten ermöglicht. Die Erläuterungen zu der vorherigen Ausführungsform mit einem Arbeitsabschnitt lassen sich in entsprechender Weise auf diese zweite Ausführungsform übertragen. Technische Lehren, die sich auf einen Arbeitsabschnitt beziehen und nicht das Zusammenspiel beider Arbeitsabschnitten zwingend erfordern, lassen sich von der zweiten Ausführungsform auch auf die erste Ausführungsform anwenden.

Fig. 6 zeigt ein Zuführelement 64 eines endoskopischen Instruments 10 gemäß einer zweiten Ausführungsform. Das Zuführelement 64 weist einen ersten Kanal 62 und einen zweiten Kanal 62' auf. An dem ersten Kanal 62 ist eine erste Längsnut 50 ausgebildet, und an dem zweiten Kanal 62' ist eine zweite Längsnut 50' ausgebildet ist. Die Längsnuten 50, 50' sind hier vollständig bis zur Peripherie des Zuführelements 64 ausgebildet. Diese Ausgestaltung erleichtert die Herstellung des Zuführelements 64, doch können die Längsnuten 50, 50' auch in geringerer Tiefe, ausgehend vom jeweiligen Kanal 62, 62', ausgebildet werden, so dass die Längsnuten 50, 50' nicht die Zylinderfläche des Zuführelements 64 erreichen und die Zylinderfläche geschlossen ist.

Bei dieser Ansicht ist außerdem der Winkelbereich W verdeutlicht, in dem ein Einfahren des Maulstücks 24 blockiert wird. Für die Betätigung des Maulstücks wird hier in vorteilhafter Weise eine Kulissenführung aus WO 2007/144172 A1 verwendet.

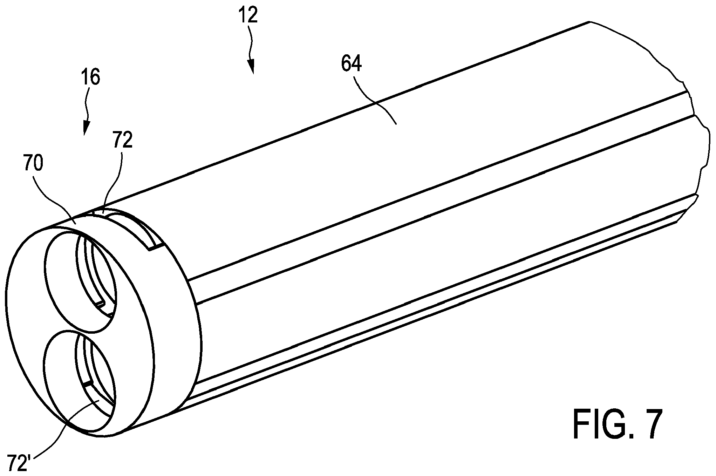

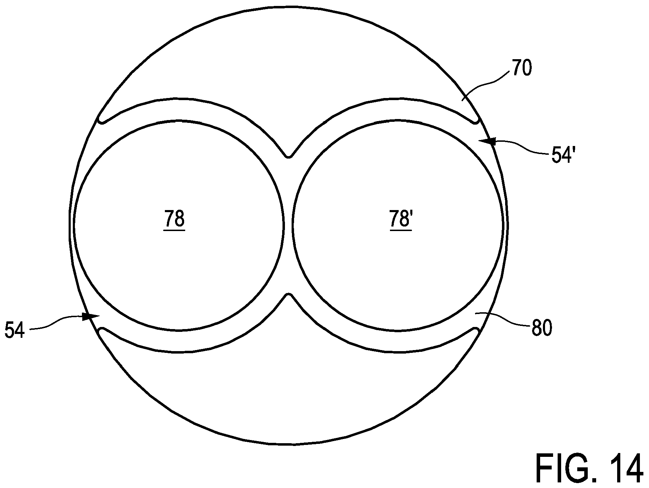

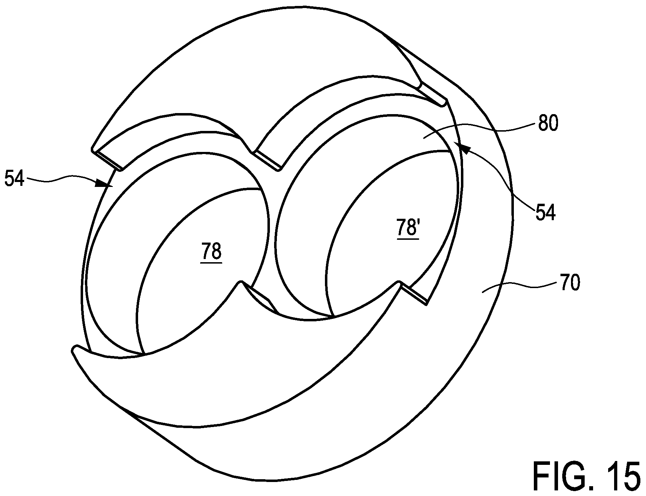

Fig. 7 zeigt das Zuführelement 64 gemäß Fig. 6 mit einem Abschlusselement 70, das im Vergleich zu dem Abschlusselement 66 aus Fig. 5 zwei Öffnungen aufweist. Das Abschlusselement 70 ist in den Figuren 14 und 15 noch deutlicher dargestellt wird. Es ist aber bereits hier zu erkennen, dass der Schaft 12 am distalen Ende 16 einen ersten Öffnungsschlitz 72 und einen zweiten Öffnungsschlitz 72' aufweist, in den ein Vorsprung 52 der Sperrvorrichtung 32 bei einer Rotation um die Längsrichtung L eintreten kann.

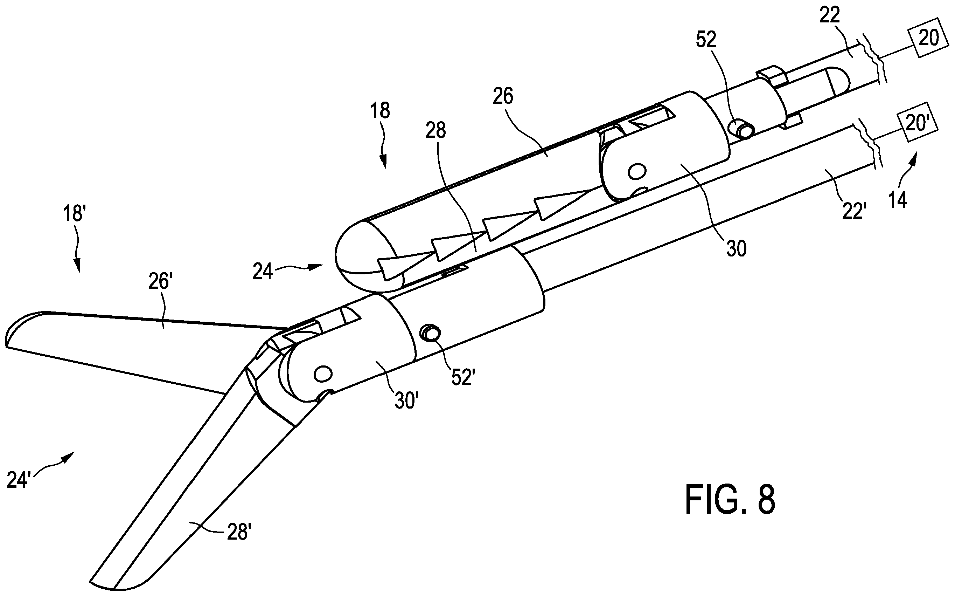

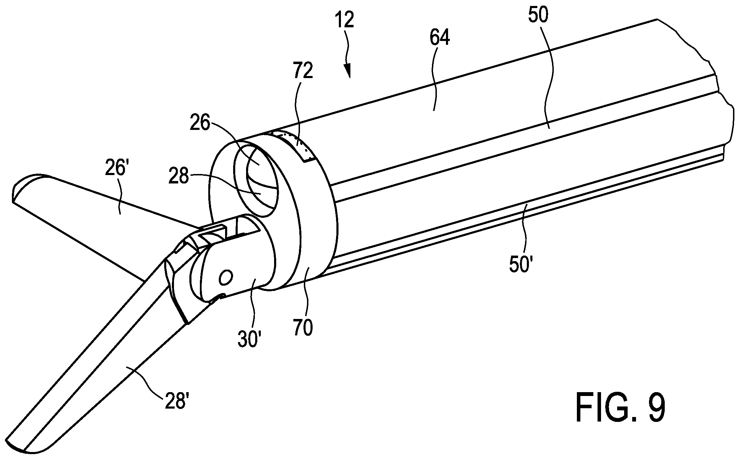

Fig. 8 zeigt in isolierter Darstellung, dass zusätzlich zu dem ersten Arbeitsabschnitt 18' ein zweiter Arbeitsabschnitt 18' vorhanden ist. Außerdem ist am proximalen Ende zusätzlich zum ersten Betätigungselement 20 ein zweites Betätigungselement 20' angeordnet. Das zweite Betätigungselement 20' ermöglicht eine Betätigung des zweiten Arbeitsabschnitts 18'. Der zweite Arbeitsabschnitt 18' ist mit dem zweiten Betätigungselement 20' über ein zweites Schubelement 22' wirkverbunden, das sich entlang der Längsrichtung L erstreckt. Der zweite Arbeitsabschnitt 18' weist ein zweites Maulstück 24' mit einem dritten Maulteil 26' und einem vierten Maulteil 28' auf. Das zweite Maulstück 24' ist an einem zweiten Maulteilhalter 30' verschwenkbar angeordnet. Der zweite Maulteilhalter 30' ist im Schaft 12 (siehe Fig. 9) entlang der Längsrichtung L verschiebbar und um die Längsrichtung L rotierbar angeordnet.

Das zweite Schubelement 22' ist mit dem zweiten Maulstück 24' gekoppelt. Das zweite Maulstück 24' ist derart ausgebildet, dass es im geschlossenen Zustand in den Schaft 12 einfahrbar ist, insbesondere in den zweiten Kanal 62' des Zuführelements 64 (siehe Fig. 6). Innerhalb des Instruments 10 ist eine zweite Sperrvorrichtung 32' angeordnet, die dafür ausgebildet ist, ein Einfahren des zweiten Maulstücks 24' in den Schaft 12 in einem auf die Längsrichtung L bezogenen zweiten Winkelbereich W' zu blockieren. Die Erläuterungen zum ersten Winkelbereich W gelten auch für den zweiten Winkelbereich W'. Bei dieser Ausgestaltung sind die Winkelbereiche gleich gewählt. Sie können sich bei bevorzugten Ausführungsformen aber auch unterscheiden.

Fig. 9 zeigt, wie die Arbeitsabschnitte aus Fig. 8 in den Aufbau gemäß Fig. 7 eingesetzt sind.

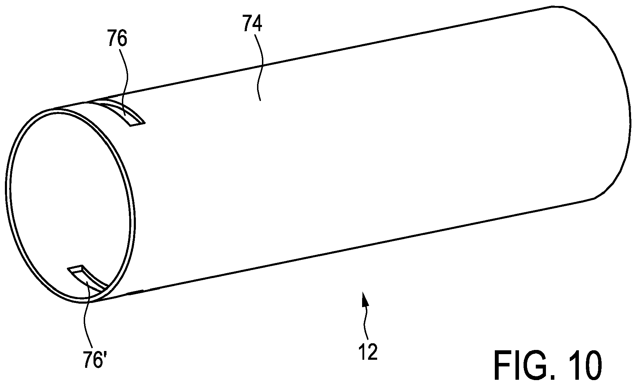

Fig. 10 zeigt eine Hülle 74 des Schafts 12. Auch hier sind Öffnungsschlitze 76, 76' ausgebildet, die mit den Öffnungsschlitzen 72, 72' korrespondieren und auch das Eintreten eines Vorsprungs 52 ermöglichen. Die Hülle 74 dient der Verbesserung der Fertig- und Anwendbarkeit und ist nicht zwingend erforderlich.

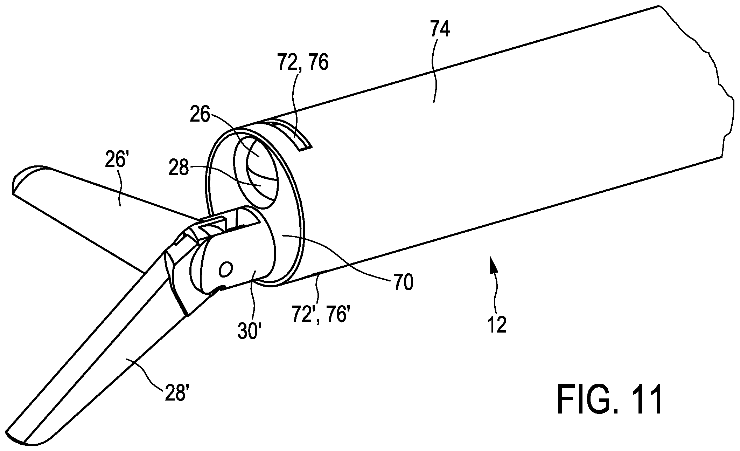

Fig. 11 zeigt die Darstellung aus Fig. 9, wenn die Hülle 74 gemäß Fig. 10 aufgesetzt ist.

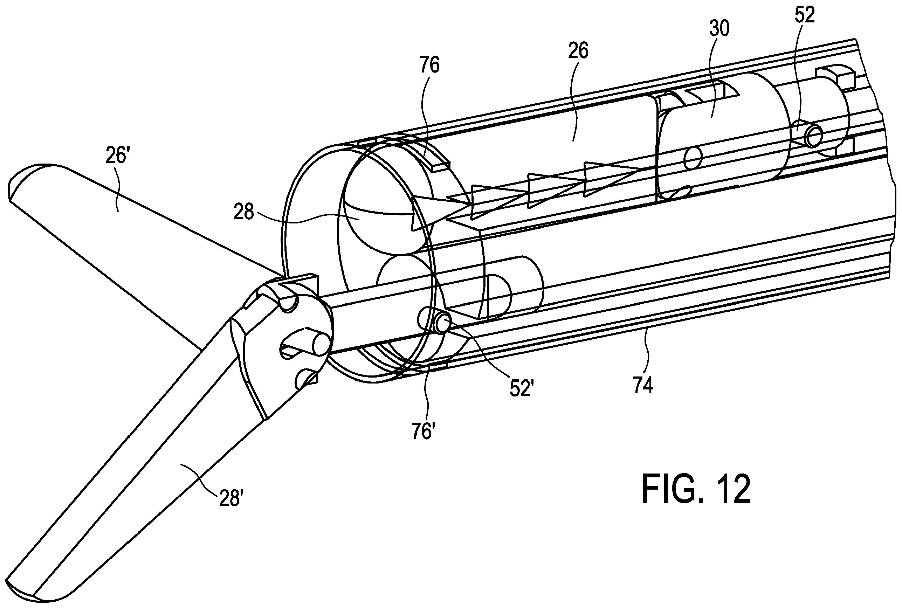

Fig. 12 zeigt eine teiltransparente Darstellung der Fig. 11, in der das Abschlusselement 66 nicht gezeigt ist.

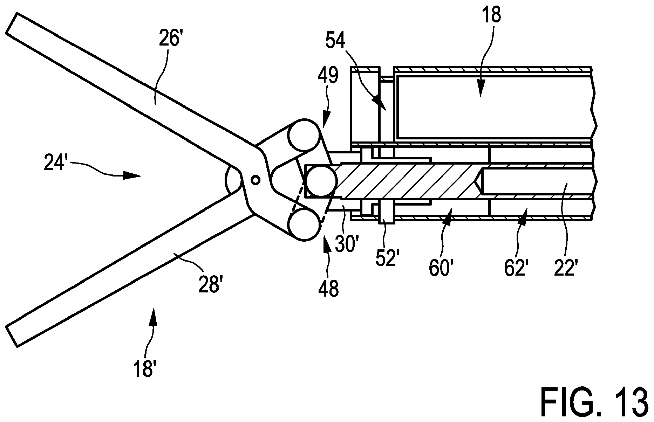

Fig. 13 zeigt eine vereinfachte Ansicht des Inneren des distalen Teils der zweiten Ausführungsform. Es ist hier zu erkennen, dass der zweite Vorsprung 52' durch die Öffnungsschlitze 72', 76' teilweise ausgetreten ist.

Es ist zu sehen, dass eine Verlagerung des zweiten Schubelements 22' in Längsrichtung L nun das Maulstück 24' öffnet oder schließt. Eine Verlagerung des zweiten Maulteilhalters 30' ist in der gezeigten Darstellung nicht möglich, da der zweite Vorsprung 52' nicht mit der zweiten Längsnut 50' korrespondiert. Wird der zweite Maulteilhalter 30' mittels des zweiten Schubelements 22' so gedreht, dass der zweite Vorsprung 52' mit der zweiten Längsnut 50' korrespondiert, führt ein Zug am Schubelement 22' zu einem Schließen des zweiten Maulstücks 24' und schließlich zu einem Einfahren des zweiten Maulstücks 24'.

Fig. 14 zeigt eine erste Ausführungsform des Abschlusselements 70. Es sind die Öffnungen 78 und 78' für den ersten bzw. zweiten Arbeitsabschnitt 18, 18' zu erkennen. Außerdem ist ein vertiefter Bereich 80 zu erkennen, der die Ringnut 54 bzw. 54' für den Vorsprung 52 bzw. 52' bildet.

Fig. 15 zeigt das Abschlusselement 70 in perspektivischer Darstellung.

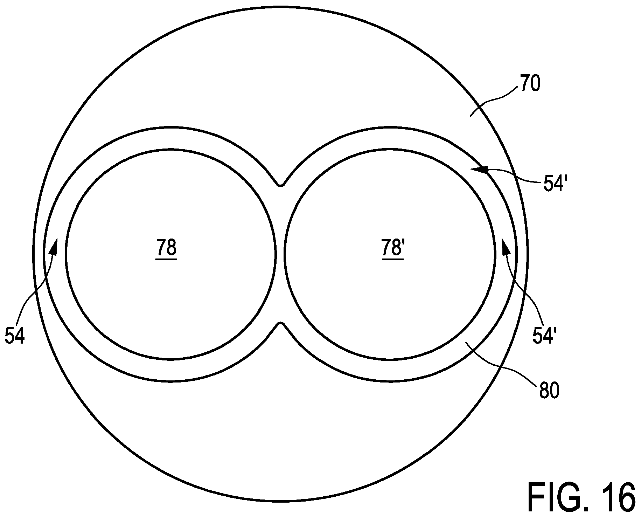

Fig. 16 zeigt eine zweite Ausführungsform eines Abschlusselements 70. Im Vergleich zur Fig. 14 sind die Dimensionen hier so gewählt, dass die Öffnungen 78, 78' denselben Durchmesser haben, das Abschlusselement 70 insgesamt aber einen größeren Durchmesser hat. Dies ermöglicht es, dass die Vorsprünge 52, 52' vollständig innerhalb des Schafts 12 geführt werden und zumindest auf die äußeren Öffnungsschlitze 76, 76' verzichtet werden kann.

Es wurde ein endoskopisches Instrument 10 aufgezeigt, das selbst bei geringem Durchmesser und großer Länge des Schafts vielfältige Bewegungsmöglichkeiten des Arbeitsabschnitts am distalen Ende des Instruments ermöglicht.

## Patentansprüche

1. Endoskopisches Instrument (10) mit einem Schaft (12), der sich zwischen einem proximalen Ende (14) des Instruments (10) und einem distalen Ende (16) des Instruments (10) entlang einer Längsrichtung (L) erstreckt, wobei am distalen Ende (16) ein Arbeitsabschnitt (18) angeordnet ist und am proximalen Ende (14) ein Betätigungselement (20) angeordnet ist, das eine Betätigung des Arbeitsabschnitts (18) ermöglicht, wobei der Arbeitsabschnitt (18) und das Betätigungselement (20) über ein Schubelement (22) wirkverbunden sind, das sich entlang der Längsrichtung (L) erstreckt, wobei der Arbeitsabschnitt (18) ein Maulstück (24) mit einem ersten Maulteil (26) und einem zweiten Maulteil (28) aufweist, das an einem Maulteilhalter (30) verschwenkbar angeordnet ist, wobei der Maulteilhalter (30) im Schaft (12) entlang der Längsrichtung (L) verschiebbar und um die Längsrichtung (L) rotierbar angeordnet ist und das Schubelement (22) mit dem Maulstück (24) gekoppelt ist, wobei das Maulstück (24) derart ausgebildet ist, dass es im geschlossenen Zustand in den Schaft (12) einfahrbar ist, wobei in dem Instrument (10) eine Sperrvorrichtung (32) angeordnet ist, die dafür ausgebildet ist, ein Einfahren des Maulstücks (24) in den Schaft (12) in einem vorgegebenen Winkelbereich (W) um die Längsrichtung (L) zu blockieren, wobei das Schubelement (22) entlang der Längsrichtung (L) zumindest dann relativ zum Maulteilhalter (30) verlagerbar ist, wenn das Maulstück (24) aus dem Schaft (12) ausgefahren ist, und wobei das Schubelement (22) bei einem Schub auf das distale Ende (16) hin eine Kraft auf das Maulstück (24) ausübt, die das Maulstück (24) öffnen kann, und der Schaft (12) räumlich so ausgebildet ist, dass ein Öffnen des Maulstücks (24) im Schaft (12) verhindert wird.

2. Endoskopisches Instrument nach Anspruch 1, wobei die Sperrvorrichtung (32) ein erstes Sperrelement aufweist, das ortsfest relativ zum Schaft (12) angeordnet ist, und ein zweites Sperrelement aufweist, das ortsfest relativ zum Maulteilhalter (30) angeordnet ist, wobei das erste Sperrelement bevorzugt als Nut und das zweite Sperrelement bevorzugt als Vorsprung (52) ausgestaltet sind.

3. Endoskopisches Instrument nach einem der vorhergehenden Ansprüche, wobei die Sperrvorrichtung (32) eine Längsnut (50) aufweist, die im Schaft (12) entlang der Längsrichtung (L) ausgebildet ist, und einen Vorsprung (52) aufweist, der am Maulteilhalter (30) angeordnet ist und bei einer Verlagerung des Maulteilhalters (30) entlang der Längsrichtung (L) in der Längsnut (50) geführt ist.

4. Endoskopisches Instrument nach einem der vorhergehenden Ansprüche, wobei am distalen Ende des Schafts (12) eine Ringnut (54) ausgebildet ist, in die ein am Maulteilhalter (30) angeordneter Vorsprung (52) eintreten kann, so dass der Maulteilhalter (30) um die Längsrichtung (L) rotiert werden kann, wenn der Vorsprung (52) in die Ringnut (54) eingetreten ist.

5. Endoskopisches Instrument nach einem der vorhergehenden Ansprüche, wobei die Sperrvorrichtung (32) eine Längsnut (50) aufweist, die im Schaft (12) entlang der Längsrichtung (L) ausgebildet ist, eine Ringnut (54) am distalen Ende des Schafts (12) aufweist und einen am Maulteilhalter (30) angeordneten Vorsprung (52), wobei der Vorsprung (52) in der Längsnut (50) und in der Ringnut (54) geführt ist.

6. Endoskopisches Instrument nach einem der vorhergehenden Ansprüche, wobei das erste Maulteil (26) und das zweite Maulteil (28) um eine gemeinsame erste Achse (44) verschwenkbar sind und das Maulstück (24) an einer zweiten Achse (46) mit dem Schubelement (22) gekoppelt ist, wobei die erste Achse (44) und die zweite Achse (46) voneinander beabstandet sind.

7. Endoskopisches Instrument nach einem der vorhergehenden Ansprüche, wobei das erste Maulteil (26) über eine erste gelenkige Verbindung (48) mit dem Schubelement (22) gekoppelt ist und/oder das zweite Maulteil (28) über eine zweite gelenkige Verbindung (49) mit dem Schubelement (22) gekoppelt ist.

8. Endoskopisches Instrument nach einem der vorhergehenden Ansprüche, wobei am distalen Ende des Schafts (12) ein Anschlag (56) ausgebildet ist, der eine Verlagerung des Maulteilhalters (30) entlang der Längsrichtung (L) distalseitig begrenzt.

9. Endoskopisches Instrument nach einem der vorhergehenden Ansprüche, wobei das Schubelement (22) einen länglichen Schubschaft (58) aufweist, der in einer Hülse (60) des Maulteilhalters (30) verschiebbar geführt ist, wobei die Hülse (60) in einem Kanal (62) im Inneren des Schafts (12) geführt ist.

10. Endoskopisches Instrument nach einem der vorhergehenden Ansprüche, wobei der Schaft (12) am distalen Ende (16) einen Bereich (34) aufweist, der abwinkelbar ist, und die Abwinklung von einem Abwinklungssteuerelement (36) gesteuert wird, das vom proximalen Ende (14) des Instruments (10) betätigbar ist.

11. Endoskopisches Instrument nach einem der vorhergehenden Ansprüche, wobei am distalen Ende (16) ein zweiter Arbeitsabschnitt (18') angeordnet ist und am proximalen Ende (14) ein zweites Betätigungselement angeordnet ist, das eine Betätigung des zweiten Arbeitsabschnitts (18') ermöglicht, wobei der zweite Arbeitsabschnitt (18') und das zweite Betätigungselement (20') über ein zweites Schubelement (22') wirkverbunden sind, das sich entlang der Längsrichtung (L) erstreckt, wobei der zweite Arbeitsabschnitt (18') ein zweites Maulstück (24') mit einem dritten Maulteil (26') und einem vierten Maulteil (28') aufweist, das an einem zweiten Maulteilhalter (30') verschwenkbar angeordnet sind, wobei der zweite Maulteilhalter (30') im Schaft (12) entlang der Längsrichtung (L) verschiebbar und um die Längsrichtung (L) rotierbar angeordnet ist und das zweite Schubelement (22') mit dem zweiten Maulstück (24') gekoppelt ist, wobei das zweite Maulstück (24') derart ausgebildet ist, dass es im geschlossenen Zustand in den Schaft (12) einfahrbar ist, wobei innerhalb des Instruments (10) eine zweite Sperrvorrichtung (32') angeordnet ist, die dafür ausgebildet ist, ein Einfahren des zweiten Maulstücks (24') in den Schaft (12) in einem zweiten Winkelbereich (W') um die Längsrichtung (L) zu blockieren.

12. Endoskopisches Instrument nach einem der vorhergehenden Ansprüche, wobei der Schaft (12) am distalen Ende (16) einen Öffnungsschlitz (72) aufweist, in den ein Vorsprung (52) der Sperrvorrichtung (32) bei einer Rotation um die Längsrichtung (L) eintreten kann.

13. Endoskopisches Instrument nach einem der vorhergehenden Ansprüche, wobei der Winkelbereich (W) mindestens 180° beträgt.

## Claims

1. Endoscopic instrument (10) having a shaft (12) which extends between a proximal end (14) of the instrument (10) and a distal end (16) of the instrument (10) along a longitudinal direction (L), wherein a working section (18) is arranged on the distal end (16) and an actuating element (20) is arranged on the proximal end (14), which actuating element permits an actuation of the working section (18), wherein the working section (18) and the actuating element (20) are operatively connected by a thrust element (22) which extends along the longitudinal direction (L), wherein the working section (18) has a jaw piece (24) with a first jaw part (26) and a second jaw part (28), which jaw piece is arranged pivotably on a jaw part holder (30), wherein the jaw part holder (30) is arranged in the shaft (12) so as to be displaceable along the longitudinal direction (L) and rotatable about the longitudinal direction (L) and the thrust element (22) is coupled to the jaw piece (24), wherein the jaw piece (24) is configured such that, in the closed state, it can be retracted into the shaft (12), wherein, in the instrument (10), there is provided a blocking device (32) which is configured to block a retraction of the jaw piece (24) into the shaft (12) in a predefined angle range (W) about the longitudinal direction (L), wherein the thrust element (22) is displaceable along the longitudinal direction (L) relative to the jaw part holder (30) at least when the jaw piece (24) has been deployed out of the shaft (12), and wherein the thrust element (22), when subjected to a thrust action onto the distal end (16), exerts a force on the jaw piece (24) which can open the jaw piece (24), and the shaft (12) is spatially configured such that an opening of the jaw piece (24) in the shaft (12) is prevented.

2. Endoscopic instrument of claim 1, wherein the blocking device (32) has a first blocking element, which is arranged positionally fixedly relative to the shaft (12), and a second blocking element, which is arranged positionally fixedly relative to the jaw part holder (30), wherein the first blocking element is preferably in the form of a groove, and the second blocking element is preferably in the form of a projection (52).

3. Endoscopic instrument of any preceding claim, wherein the blocking device (30) has a longitudinal groove (50), which is formed in the shaft (12) along the longitudinal direction (L), and has a projection (52), which is arranged on the jaw part holder (30) and which is guided in the longitudinal groove (50) during a displacement of the jaw part holder (30) along the longitudinal direction (L).

4. Endoscopic instrument of any preceding claim, wherein, on the distal end of the shaft (12), there is formed an annular groove (54) into which a projection (52) arranged on the jaw part holder (30) can enter, such that the jaw part holder (30) can be rotated about the longitudinal direction (L) when the projection (52) has entered the annular groove (54).

5. Endoscopic instrument of any preceding claim, wherein the blocking device (32) has a longitudinal groove (50), which is formed in the shaft (12) along the longitudinal direction (L), has an annular groove (54) on the distal end of the shaft (12), and has a projection (52) arranged on the jaw part holder (30), wherein the projection (52) is guided in the longitudinal groove (50) and in the annular groove (54).

6. Endoscopic instrument of any preceding claim, wherein the first jaw part (26) and the second jaw part (28) are pivotable about a common first axis (44) and the jaw piece (24) is coupled to the thrust element (22) at a second axis (46), wherein the first axis (44) and the second axis (46) are spaced apart from one another.

7. Endoscopic instrument of any preceding claim, wherein the first jaw part (26) is coupled to the thrust element (22) by way of a first articulated connection (48) and/or the second jaw part (28) is coupled to the thrust element (22) by way of a second articulated connection (49).

8. Endoscopic instrument of any preceding claim, wherein, on the distal end of the shaft (12), there is formed a stop (56) which delimits a displacement of the jaw part holder (30) along the longitudinal direction (L) at the distal side.

9. Endoscopic instrument of any preceding claim, wherein the thrust element (22) has an elongate thrust shaft (58) which is guided displaceably in a sleeve (60) of the jaw part holder (30), wherein the sleeve (60) is guided in a channel (62) in the interior of the shaft (12).

10. Endoscopic instrument of any preceding claim, wherein the shaft (12) has, at the distal end (16), a region (34) which can be angularly deflected, and the angular deflection is controlled by an angular-deflection control element (36) which can be actuated from the proximal end (14) of the instrument (10).

11. Endoscopic instrument of any preceding claim, wherein a second working section (18') is arranged on the distal end (16) and a second actuating element is arranged on the proximal end (14), which second actuating element permits an actuation of the second working section (18'), wherein the second working section (18') and the second actuating element (20') are operatively connected by way of a second thrust element (22') which extends along the longitudinal direction (L), wherein the second working section (18') has a second jaw piece (24') with a third jaw part (26') and with a fourth jaw part (28'), which second jaw piece is pivotably arranged on a second jaw part holder (30'), wherein the second jaw part holder (30') is arranged in the shaft (12) so as to be displaceable along the longitudinal direction (L) and rotatable about the longitudinal direction (L), and the second thrust element (22') is coupled to the second jaw piece (24'), wherein the second jaw piece (24') is configured such that, in the closed state, it can be retracted into the shaft (12), wherein, within the instrument (10), there is arranged a second blocking device (32') which is configured to block a retraction of the second jaw piece (24') into the shaft (12) in a second angle range (W') about the longitudinal direction (L).

12. Endoscopic instrument of any preceding claim, wherein the shaft (12) has, on the distal end (16), an opening slot (72) into which a projection (52) of the blocking device (32) can enter during a rotation about the longitudinal direction (L).

13. Endoscopic instrument of any preceding claim, wherein the angle range (W) is at least 180°.

## Revendications

1. Instrument endoscopique (10) comprenant une tige (12) qui s'étend entre une extrémité proximale (14) de l'instrument (10) et une extrémité distale (16) de l'instrument (10) le long d'une direction longitudinale (L), une portion de travail (18) étant disposée à l'extrémité distale (16) et un élément d'actionnement (20) étant disposé à l'extrémité proximale (14), lequel permet un actionnement de la portion de travail (18), la portion de travail (18) et l'élément d'actionnement (20) étant en liaison fonctionnelle par le biais d'un élément de poussée (22) qui s'étend le long de la direction longitudinale (L), la portion de travail (18) présentant une pièce formant mâchoire (24) avec une première partie de mâchoire (26) et une deuxième partie de mâchoire (28), qui est disposée de manière à pouvoir pivoter sur un support de partie de mâchoire (30), le support de partie de mâchoire (30) étant disposé de manière à pouvoir être déplacé dans la tige (12) le long de la direction longitudinale (L) et étant disposé de manière à pouvoir tourner autour de la direction longitudinale (L) et l'élément de poussée (22) étant accouplé à la pièce formant mâchoire (24), la pièce formant mâchoire (24) étant réalisée de telle sorte que dans l'état fermé, elle puisse être introduite dans la tige (12), un dispositif de blocage (32) étant disposé dans l'instrument (10), lequel est réalisé de manière à bloquer une introduction de la pièce formant mâchoire (24) dans la tige (12) dans une plage angulaire prédéfinie (W) autour de la direction longitudinale (L), l'élément de poussée (22) pouvant alors être déplacé le long de la direction longitudinale (L) au moins relativement par rapport au support de partie de mâchoire (30) lorsque la pièce formant mâchoire (24) est sortie de la tige (12), et l'élément de poussée (22), lors d'une poussée sur l'extrémité distale (16), exerçant une force sur la pièce formant mâchoire (24) qui peut ouvrir la pièce formant mâchoire (24), et la tige (12) étant réalisée spatialement de telle sorte qu'une ouverture de la pièce formant mâchoire (24) dans la tige (12) soit évitée.

2. Instrument endoscopique selon la revendication 1, dans lequel le dispositif de blocage (32) présente un premier élément de blocage qui est disposé fixement par rapport à la tige (12), et présente un deuxième élément de blocage qui est disposé fixement par rapport au support de partie de mâchoire (30), le premier élément de blocage étant configuré de préférence sous forme de rainure et le deuxième élément de blocage étant configuré de préférence sous forme de saillie (52).

3. Instrument endoscopique selon l'une quelconque des revendications précédentes, dans lequel le dispositif de blocage (32) présente une rainure longitudinale (50) qui est réalisée dans la tige (12) le long de la direction longitudinale (L) et présente une saillie (52) qui est disposée sur le support de partie de mâchoire (30) et qui est guidée dans la rainure longitudinale (50) dans le cas d'un déplacement du support de partie de mâchoire (30) le long de la direction longitudinale (L).

4. Instrument endoscopique selon l'une quelconque des revendications précédentes, dans lequel à l'extrémité distale de la tige (12) est réalisée une rainure annulaire (54) dans laquelle peut entrer une saillie (52) disposée au niveau du support de partie de mâchoire (30), de telle sorte que le support de partie de mâchoire (30) puisse être tourné autour de la direction longitudinale (L) lorsque la saillie (52) est entrée dans la rainure annulaire (54).

5. Instrument endoscopique selon l'une quelconque des revendications précédentes, dans lequel le dispositif de blocage (32) présente une rainure longitudinale (50) qui est réalisée dans la tige (12) le long de la direction longitudinale (L), présente une rainure annulaire (54) à l'extrémité distale de la tige (12) et une saillie (52) disposée au niveau du support de partie de mâchoire (30), la saillie (52) étant guidée dans la rainure longitudinale (50) et dans la rainure annulaire (54).

6. Instrument endoscopique selon l'une quelconque des revendications précédentes, dans lequel la première partie de mâchoire (26) et la deuxième partie de mâchoire (28) peuvent pivoter autour d'un premier axe commun (44) et la pièce formant mâchoire (24) est accouplée au niveau d'un deuxième axe (46) à l'élément de poussée (22), le premier axe (44) et le deuxième axe (46) étant espacés l'un de l'autre.

7. Instrument endoscopique selon l'une quelconque des revendications précédentes, dans lequel la première partie de mâchoire (26) est accouplée par le biais d'une première liaison articulée (48) à l'élément de poussée (22) et/ou la deuxième partie de mâchoire (28) est accouplée par le biais d'une deuxième liaison articulée (49) à l'élément de poussée (22).

8. Instrument endoscopique selon l'une quelconque des revendications précédentes, dans lequel à l'extrémité distale de la tige (12) est réalisée une butée (56) qui limite du côté distal un déplacement du support de partie de mâchoire (30) le long de la direction longitudinale (L).

9. Instrument endoscopique selon l'une quelconque des revendications précédentes, dans lequel l'élément de poussée (22) présente une tige de poussée allongée (58) qui est guidée de manière déplaçable dans un manchon (60) du support de partie de mâchoire (30), le manchon (60) étant guidé dans un canal (62) à l'intérieur de la tige (12).

10. Instrument endoscopique selon l'une quelconque des revendications précédentes, dans lequel la tige (12) présente à l'extrémité distale (16) une région (34) qui peut être fléchis et la flexion est commandée par un élément de commande de flexion (36) qui peut être actionné par l'extrémité proximale (14) de l'instrument (10).

11. Instrument endoscopique selon l'une quelconque des revendications précédentes, dans lequel, à l'extrémité distale (16), est disposée une deuxième portion de travail (18') et à l'extrémité proximale (14), est disposé un deuxième élément d'actionnement, qui permet un actionnement de la deuxième portion de travail (18'), la deuxième portion de travail (18') et le deuxième élément d'actionnement (20') étant en liaison fonctionnelle par le biais d'un deuxième élément de poussée (22') qui s'étend le long de la direction longitudinale (L), la deuxième portion de travail (18') présentant une deuxième pièce formant mâchoire (24') avec une troisième partie de mâchoire (26') et une quatrième partie de mâchoire (28'), lesquelles sont disposées de manière à pouvoir pivoter sur un deuxième support de partie de mâchoire (30'), le deuxième support de partie de mâchoire (30') étant disposé de manière à pouvoir être déplacé dans la tige (12) le long de la direction longitudinale (L) et étant disposé de manière à pouvoir tourner autour de la direction longitudinale (L) et le deuxième élément de poussée (22') étant accouplé à la deuxième pièce formant mâchoire (24'), la deuxième pièce formant mâchoire (24') étant réalisée de telle sorte que dans l'état fermé, elle puisse être introduite dans la tige (12), un deuxième dispositif de blocage (32') étant disposé à l'intérieur de l'instrument (10), lequel est réalisé de manière à bloquer une introduction de la deuxième pièce formant mâchoire (24') dans la tige (12) dans une deuxième plage angulaire (W') autour de la direction longitudinale (L).

12. Instrument endoscopique selon l'une quelconque des revendications précédentes, dans lequel la tige (12) à l'extrémité distale (16) présente une fente d'ouverture (72) dans laquelle peut pénétrer une saillie (52) du dispositif de blocage (32) dans le cas d'une rotation autour de la direction longitudinale (L).

13. Instrument endoscopique selon l'une quelconque des revendications précédentes, dans lequel la plage angulaire (W) vaut au moins 180°.
